## Europäisches Patentamt
## European Patent Office
## Office européen des brevets

(11) Veröffentlichungsnummer: **0 254 765**
**B1**

(12) **EUROPÄISCHE PATENTSCHRIFT**

(45) Veröffentlichungstag der Patentschrift:
18.10.89

(51) Int. Cl.⁴: **A61M 5/315**

(21) Anmeldenummer: **86110644.1**

(22) Anmeldetag: **01.08.86**

(54) Spritze für medizinische Zwecke.

(43) Veröffentlichungstag der Anmeldung:
**03.02.88 Patentblatt 88/5**

(45) Bekanntmachung des Hinweises auf die Patenterteilung:
**18.10.89 Patentblatt 89/42**

(84) Benannte Vertragsstaaten:
**AT BE CH DE FR GB IT LI LU NL SE**

(56) Entgegenhaltungen:
**FR-A- 1 517 960**

(73) Patentinhaber: **Vetter, Helmut, Marienplatz 81, D-7980 Ravensburg(DE)**

(72) Erfinder: **Geprägs, Peter, Judithaweg 6, D-7987 Weingarten(DE)**
Erfinder: **Frasch, Eugen, Ludwig-Thoma-Weg 2, D-7991 Oberteuringen(DE)**

(74) Vertreter: **Fay, Hermann, Dipl.-Phys. Dr., Ensingerstrasse 21 Postfach 1767, D-7900 Ulm (Donau)(DE)**

## Beschreibung

Die Erfindung betrifft eine Spritze für medizinische Zwecke mit einem am einen Ende zum Ansatz einer Injektionsnadel ausgebildeten Spritzenzylinder und mit einem im Spritzenzylinder verschiebbaren Spritzenkolben, der fest an einer Kolbenstange sitzt, die an dem dem Nadelansatzende entgegen gesetzten Zylinderende aus dem Spritzenzylinder hervorsteht, sowie mit einem weiteren, im Spritzenzylinder auf der dem Nadelansatzende abgewandten Seite des Spritzenkolbens angeordneten, von der Kolbenstange durchgriffenen und außer im Spritzenzylinder auch auf der Kolbenstange verschiebbaren Spritzenkolben, wobei die Kolbenstange einen Längskanal aufweist, der innerhalb der Kolbenstange über den Verschiebungsbereich des auf der Kolbenstange verschiebbaren Spritzenkolbens verläuft und einerseits innerhalb des Spritzenzylinders zwischen beiden Spritzenkolben nahe dem an der Kolbenstange festen Spritzenkolben und andererseits im Außenraum des Spritzenzylinders jenseits des auf der Kolbenstange verschiebbaren Spritzenkolbens mündet.

Aus der FR-A 517 960 ist eine Doppelkammerspritze bekannt, deren eine, nadelseitige Kammer mit dem eigentlichen Wirkstoff in trockener bzw. getrockneter Form vorgefüllt ist und deren zweite Kammer das Lösungsmittel enthält, mit dem der Wirkstoff vor der Applikation in eine injektionsfähige gelöste Form gebracht wird. Bei der Befüllung dieser Doppelkammerspritze wird zunächst der Wirkstoff eingebracht, sodann der erste, die beiden Kammern trennende Spritzenkolben mit der Kolbenstange in den Spritzenzylinder eingeführt und anschließend der den Spritzenzylinder endseitig schließende zweite Spritzenkolben gesetzt. Schließlich wird die zweite Kammer mittels eines in den Längskanal der Kolbenstange eingeführten dünnen Rohres mit dem Lösungsmittel befüllt und dann der Längskanal an seinem Ende mit einem Stopfen geschlossen.

Häufig besteht jedoch der Wunsch, die Doppelkammerspritze ohne das Lösungsmittel in den Verkehr zu bringen, insbes. dann, wenn aus anderen Gründen keine Notwendigkeit gegeben ist, die Spritze von Anfang an auch mit dem Lösungsmittel zu befüllen. Dies ist in der Regel dann der Fall, wenn als Lösungsmittel destilliertes Wasser dient, das jedenfalls bei stationärer Anwendung üblicherweise jederzeit verfügbar ist. Dann nämlich läßt sich bei den konfektionierten Fertigspritzen neben einer Gewichtseinsparung auch eine geringere Baulänge des Spritzenzylinders erreichen.

Der Erfindung liegt die Aufgabe zugrunde, eine Spritze der eingangs genannten Art so auszubilden, daß sie zur Herstellung von Substanzmischungen unmittelbar vor der Injektion durch Aufziehen des Lösungsmittels geeignet ist und also einen Spritzenzylinder mit optimal kurzer Länge besitzen kann, wobei auch nach schon längere Zeit zurückliegender Sterilisation die Gefahr einer Kontamination ausgeschlossen sein soll.

Diese Aufgabe wird nach der Erfindung dadurch gelöst, daß der Längskanal durch ein Rückschlagventil sperrbar ist, das den Längskanal nur in der Richtung vom Zwischenraum zwischen beiden Spritzenkolben zum Außenraum des Spritzenzylinders gegen eine Federkraft öffnet.

Bei der Sterilisation und der Vorfüllung der erfindungsgemäßen Spritze mit der ersten Substanz befindet sich der an der Kolbenstange feste Spritzenkolben in einer nur wenig im Spritzenzylinder zurückverschobenen Position, so daß der Raum im Spritzenzylinder zwischen diesem Spritzenkolben und dem nadelansatzseitigen Zylinderende zur Aufnahme dieser Substanz ausreicht. Der zweite Spritzenkolben befindet sich dabei in seiner im Spritzenzylinder hinteren Endstellung, d. h. praktisch am hinteren Ende des Spritzenzylinders, wobei der schon genannte vordere Raum und der Zwischenraum zwischen beiden Spritzenkolben gemeinsam nur das für die fertige Injektionssubstanz benötigte Volumen aufzuweisen brauchen. Dieser Zwischenraum ist ebenfalls steril und behält seinen sterilen Zustand auch über lange Zeit bei, weil er durch den zweiten, im Spritzenzylinder hinteren Spritzenkolben zum Außenraum hin abgeschlossen ist und auch der Längskanal in der Kolbenstange durch das Rückschlagventil gesperrt ist. Wird zum Aufziehen der zweiten, flüssigen Substanz durch die Injektionsnadel hindurch der vordere Spritzenkolben im Spritzenzylinder mittels der Kolbenstange zurückgezogen, behält der zweite Spritzenkolben seine Position bei, wobei sich der Zwischenraum zwischen beiden Spritzenkolben verringert und der sich dabei in diesem Zwischenraum aufbauende Überdruck durch den Längskanal und das sich in dieser Richtung öffnende Rückschlagventil entweichen kann. Der sterile Zustand des Zwischenraums bleibt auch dabei erhalten. Um die so im Ergebnis hergestellte Injektionssubstanz zu injizieren, wird der vordere Spritzenkolben mittels der Kolbenstange im Spritzenzylinder wieder nach vorn gedrückt. Der dabei zwischen beiden Spritzenkolben entstehende Unterdruck kann sich nicht ausgleichen, weil das Rückschlagventil den Längskanal für diesen Ausgleich sperrt. Dadurch wird der hintere Spritzenkolben zwangsläufig im Spritzenzylinder mit nach vorn gezogen, so daß auch dabei der Zwischenraum nach wie vor steril bleibt. Im Ergebnis ist gewährleistet, daß der unmittelbar mit den Substanzen in Berührung kommende vordere Spritzenkolben den Spritzenzylinder über fast dessen ganze Länge rückwärts und vorwärts durchlaufen kann, ohne dabei jemals irgendwo in einen unsterilen oder bezüglich seiner Sterilität unsicheren Bereich zu gelangen.

Eine bevorzugte Ausführungsform der Erfindung ist dadurch gekennzeichnet, daß der Längskanal außenraumseitig in einer in der Kolbenstange ausgebildeten und mit einer Mündung zum Außenraum hin versehenen Ventilkammer endet, die gegenüber dem Längskanal erweitert ist und an der Mündung des Längskanals einen Ventilsitz für einen in der Ventilkammer verschiebbaren Ventilkörper bildet, der durch die Federkraft gegen den Ventilsitz gedrückt ist. Zweckmäßig ist die Ventilkammer durch ein zum Längskanal koaxiales und am freien Kolbenstangenende mündendes Sackloch gebildet, das durch einen Stopfen nach außen verschlossen ist, zwischen dem und dem Ventilkörper eine die Federkraft erzeugende Schraubenfeder eingesetzt ist. Im einzelnen soll der Ventilkörper seitlich freies Spiel gegen die Wand der Ventilkammer besitzen und die Mündung in den Außenraum vom Ventilsitz aus gesehen jenseits des Ventilkörpers vorgesehen sein. Im übrigen empfiehlt es sich, daß der Rand des außenraumseitigen Endes des Spritzenzylinders als ein den Austritt der Spritzenkolben aus dem Spritzenzylinder verhindernder Anschlag ausgebildet ist, der zugleich die hintere Endstellung des auf der Kolbenstange verschiebbaren Spritzenkolbens bestimmt.

Im folgenden wird die Erfindung an einem in der Zeichnung dargestellten Ausführungsbeispiel näher erläutert; es zeigen:

Fig. 1 eine Spritze nach der Erfindung in einem Axialschnitt,
Fig. 2 die Spritze nach Fig. 1 in voll aufgezogenem Zustand,
Fig. 3 die Spritze nach Fig. 1 in vollständig ausgedrücktem Zustand.

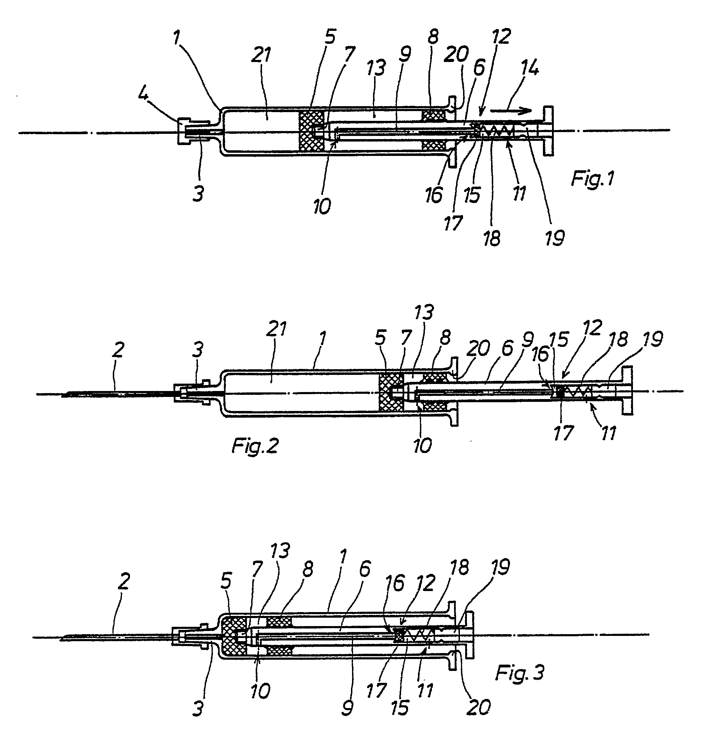

Die in der Zeichnung dargestellte Spritze besitzt einen Spritzenzylinder 1, dessen vorderes Ende zum Ansatz einer Injektionsnadel 2 ausgebildet und dazu mit einem Spritzenhals 3 versehen ist, dem entweder eine Verschlußkappe 4 wie in Fig. 1 oder die Injektionsnadel 2 wie in den Fig. 2 und 3 aufgesetzt werden kann. Im Spritzenzylinder 1 ist ein vorderer Spritzenkolben 5 verschiebbar, der fest an einer Kolbenstange 6 sitzt, die im Ausführungsbeispiel mit einem Gewindezapfen 7 in eine Gewindebohrung des Spritzenkolbens 5 eingeschraubt werden kann. Die Kolbenstange 6 steht an dem dem Nadelansatzende entgegengesetzten, zum Außenraum hin offenen Zylinderende aus dem Spritzenzylinder 1 hervor. Im Spritzenzylinder 1 ist auf der dem Nadelansatzende abgewandten Seite des ersten, vorderen Spritzenkolbens 5 ein weiterer, von der Kolbenstange 6 durchgriffener und außer im Spritzenzylinder 1 auch auf der Kolbenstange 6 selbst verschiebbarer Spritzenkolben 8 angeordnet. Die Kolbenstange 6 ist mit einem Längskanal 9 versehen, der innerhalb der Kolbenstange 6 mindestens über die Länge des Verschiebungsbereiches des auf der Kolbenstange 6 verschiebbaren hinteren Spritzenkolbens 8 verläuft. Dieser Längskanal 9 mündet einerseits bei 10 innerhalb des Spritzenzylinders 1 zwischen beiden Spritzenkolben 5, 8 nahe dem an der Kolbenstange 6 festen vorderen Spritzenkolben 5. Andererseits mündet der Längskanal 9 bei 11 im Außenraum des Spritzenzylinders 1 jenseits des auf der Kolbenstange 6 verschiebbaren Spritzenkolbens 8. Der Längskanal 9 ist normalerweise durch ein Rückschlagventil 12 gesperrt, das den Längskanal 9 nur in der Richtung vom Zwischenraum 13 zwischen beiden Spritzenkolben 5, 8 zum Außenraum des Spritzenzylinders 1 hin gegen eine Federkraft öffnet. Diese Öffnungsrichtung des Rückschlagventils 12 ist in der Fig. 1 durch den Pfeil 14 angedeutet.

Im einzelnen endet der Längskanal 9 außenraumseitig in einer in der Kolbenstange 6 ausgebildeten und mit einer Mündung 11 zum Außenraum hin versehenen Ventilkammer 15. Diese Ventilkammer 15 ist gegenüber dem Längskanal 9 erweitert. An der Mündung des Längskanals 9 in die Ventilkammer 15 ist ein Ventilsitz 16 für einen in der Ventilkammer 15 verschiebbar geführten Ventilkörper 17 gebildet, der durch die Kraft einer Feder 18 gegen den Ventilsitz 16 gedrückt ist. Diese Ventilkammer 15 ist durch ein zum Längskanal 9 koaxiales und am freien Kolbenstangenende mündendes Sackloch gebildet, das durch einen eingesetzten Stopfen 19 nach außen verschlossen ist. Zwischen diesem Stopfen 19 und dem Ventilkörper 17 befindet sich die Schraubenfeder 18, die einerseits unmittelbar am Ventilkörper 17, andererseits am Stopfen 19 abgestützt ist. Der Ventilkörper 17 besitzt seitlich zur Wand der Ventilkammer 15 hin freies Spiel, so daß sich zwischen ihm und der Wand hindurch ein Druckausgleich vollziehen kann, wenn der Ventilkörper 17 von dem Ventilsitz 16 abgehoben ist. Die Mündung 11 der Ventilkammer 15 in den Außenraum kann sich dann vom Ventilsitz 16 aus gesehen jenseits des Ventilkörpers 17 befinden. Damit der auf der Kolbenstange 6 verschiebbare, hintere Spritzenkolben 8 nicht nach hinten aus dem Spritzenzylinder 1 austreten kann, ist der Rand dieses außenraumseitigen Endes des Spritzenzylinders 1 als ein den Austritt des Spritzenkolbens 8 verhindernder Anschlag 20, nämlich in Form eines Innenwulstes, ausgebildet.

In Fig. 1 befindet sich die Spritze im Ausgangszustand. Die vordere Kammer 21 ist unter sterilen Bedingungen mit einer ersten, nicht dargestellten Substanz befüllt worden. Dabei wurden die beiden Spritzenkolben 5, 8 in der aus Fig. 1 ersichtlichen Weise gesetzt. Anschließend wurde die silikonisierte und komplett mit dem Rückschlagventil 12 vormontierte Kolbenstange 6 durch die Bohrung des hinteren Spritzenkolbens 8 geschoben und in den vorderen Spritzenkolben 5 eingedreht. - Um die Spritze anzuwenden, wird nach dem Entfernen der Verschlußkappe 4 und dem Aufsetzen der gewünschten Injektionsnadel 2 die Kolbenstange 6 entsprechend Fig. 2 im Spritzenzylinder 1 nach hinten zurückgezogen, wobei der in der Kammer 13 entstehende Überdruck sich durch den Längskanal 6 über das Rückschlagventil 12 nach außen abbauen kann, denn der Überdruck in der Kammer 13 drückt den Ventilkörper 17 gegen die Feder 18 zurück und hebt ihn vom Ventilsitz 16 ab. Die Kammer 13, in der sich dabei der vordere Spritzenkolben 5 bewegt, ist trotz dieser Entlüftung nach wie vor steril. Die von Spritzenkolben 5 bei dieser Bewegung durch die Injektionsnadel 2 aufgezogene zweite, flüssige Substanz kann daher ebenso wie der Spritzenkolben 5 selbst nicht kontaminiert werden. Soll anschließend die in der Kammer 21 gebildete fertige Injektionssubstanz wieder ausgedrückt werden, wird die Kolbenstange 6 entsprechend Fig. 3 mit dem an ihr festen vorderen Spritzenkolben 5 in die im Spritzenzylinder 1 vordere Endstellung gedrückt. Der durch diese Bewegung in der Kammer 13 entstehende Unterdruck zieht zwangsläufig den hinteren Spritzenkolben 8 mit nach vorn, weil das Rückschlagventil 12 den Ausgleich des Unterdrucks nicht erlaubt, nämlich durch den von der Feder 18 gegen den Ventilsitz 16 gedrückten Ventilkörper 17 verschlossen bleibt. Daher bleibt der Zwischenraum 13 auch bei dieser Bewegung steril und der mit der Substanz allein und unmittelbar in Berührung kommende vordere Spritzenkolben 5 bewegt sich längs seines Weges hin und zurück im Spritzenzylinder 1 ausschließlich in sterilen Bereichen.

## Patentansprüche

1. Spritze für medizinische Zwecke mit einem am einen Ende zum Ansatz einer Injektionsnadel (2) ausgebildeten Spritzenzylinder (1) und mit einem im Spritzenzylinder (1) verschiebbaren Spritzenkolben (5), der fest an einer Kolbenstange (6) sitzt, die an dem dem Nadelansatzende entgegengesetzten Zylinderende aus dem Spritzenzylinder (1) hervorsteht, sowie mit einem weiteren im Spritzenzylinder (1) auf der dem Nadelansatzende abgewandten Seite des Spritzenkolbens (5) angeordneten, von der Kolbenstange (6) durchgriffenen und außer im Spritzenzylinder (1) auch auf der Kolbenstange (6) verschiebbaren Spritzenkolben (8), wobei die Kolbenstange (6) einen Längskanal (9) aufweist, der innerhalb der Kolbenstange (6) über den Verschiebungsbereich des auf der Kolbenstange (6) verschiebbaren Spritzenkolbens (8) verläuft und einerseits innerhalb des Spritzenzylinders (1) zwischen beiden Spritzenkolben (5, 8) nahe dem an der Kolbenstange (6) festen Spritzenkolben (5) und andererseits im Außenraum des Spritzenzylinders (1) jenseits des auf der Kolbenstange (6) verschiebbaren Spritzenkolbens (8) mündet, dadurch gekennzeichnet, daß der Längskanal (9) durch ein Rückschlagventil (12) sperrbar ist, das den Längskanal (9) nur in der Richtung vom Zwischenraum (13) zwischen beiden Spritzenkolben (5, 8) zum Außenraum des Spritzenzylinders (1) gegen eine Federkraft (18) öffnet.

2. Spritze nach Anspruch 1, dadurch gekennzeichnet, daß der Längskanal (9) außenraumseitig in einer in der Kolbenstange (6) ausgebildeten und mit einer Mündung (11) zum Außenraum hin versehenen Ventilkammer (15) endet, die gegenüber dem Längskanal (9) erweitert ist und an der Mündung des Längskanals (9) einen Ventilsitz (16) für einen in der Ventilkammer (15) verschiebbaren Ventilkörper (17) bildet, der durch die Federkraft (18) gegen den Ventilsitz (16) gedrückt ist.

3. Spritze nach Anspruch 2, dadurch gekennzeichnet, daß die Ventilkammer (15) durch ein zum Längskanal (9) koaxiales und am freien Kolbenstangenende mündendes Sackloch gebildet ist, das durch einen Stopfen (19) nach außen verschlossen ist, zwischen dem und dem Ventilkörper (17) eine die Federkraft (18) erzeugende Schraubenfeder eingesetzt ist.

4. Spritze nach Anspruch 2 oder 3, dadurch gekennzeichnet, daß der Ventilkörper (17) seitlich freies Spiel gegen die Wand der Ventilkammer (15) besitzt und die Mündung (11) in den Außenraum vom Ventilsitz (16) aus gesehen jenseits des Ventilkörpers (17) vorgesehen ist.

5. Spritze nach einem der Ansprüche 1 bis 4, dadurch gekennzeichnet, daß der Rand des außenraumseitigen Endes des Spritzenzylinders (1) als ein den Austritt der Spritzenkolben (5, 8) aus dem Spritzenzylinder (1) verhindernder Anschlag (20) ausgebildet ist.

## Revendications

1. Seringue pour applications médicales comprenant un cylindre de seringue (1) conformé à l'une de ses extrémités pour l'adaptation d'une aiguille d'injection (2), un piston de seringue (5) déplaçable dans le cylindre de seringue (1) et fixé sur une tige de piston (6) laquelle dépasse du cylindre de seringue (1) à l'extrémité du cylindre opposée à l'extrémité d'adaptation de l'aiguille, ainsi qu'un piston de seringue supplémentaire (8) qui est disposé dans le cylindre de seringue (1), du côté du piston de seringue opposé à l'extrémité d'adaptation de l'aiguille et traversé par la tige de piston (6) et qui peut être déplacé non seulement dans le cylindre de seringue (1) mais aussi sur la tige de piston (6), ladite tige de piston (6) comprenant un canal longitudinal (9) qui s'étend à l'intérieur de la tige de piston (6) sur la zone de déplacement du piston de seringue (8) mobile sur la tige de piston (6) et débouche d'une part à l'intérieur du cylindre de seringue (1) entre les deux pistons de seringue (5, 8), à proximité du piston de seringue (5) solidaire de la tige de piston (6) et d'autre part à l'extérieur du cylindre de seringue (1), au-delà du piston de seringue (8) déplaçable sur la tige de piston (6), caractérisée par le fait que le canal longitudinal (9) peut être bloqué par un clapet antiretour (12) qui, sous l'action d'un ressort (18), n'ouvre le canal longitudinal (9) que dans la direction de l'espace intermédiaire (13) entre les deux pistons de seringue (5, 8) vers l'extérieur du cylindre de seringue (1).

2. Seringue selon la revendication 1, caractérisée par le fait que le canal longitudinal (9) se termine, du côté de l'extérieur, par une chambre de soupape (15) conformée dans la tige de piston (6) et munie d'une ouverture (11) vers l'extérieur, qui est élargie par rapport au canal longitudinal (9) et forme à la sortie dudit canal longitudinal (9) un siège de soupape (16) pour un corps de soupape (17) déplaçable dans la chambre de soupape (15) lequel corps de soupape est appliqué par l'action d'un ressort (18) contre ledit siège de soupape (16).

3. Seringue selon la revendication 2, caractérisée par le fait que la chambre de soupape (15) est constituée par un trou borgne coaxial au canal longitudinal (9) et débouchant à l'extrémité libre de la tige de piston, lequel trou borgne est fermé vers l'extérieur par un bouchon (19) et entre ledit bouchon et le corps de soupape (17) est inséré un ressort cylindrique (18) qui génère la force de ressort.

4. Seringue selon l'une des revendications 2 ou 3, caractérisée par le fait que le corps de soupape (17) a du jeu latéral par rapport à la paroi de la chambre de soupape (15) et que, vue à partir du siège de soupape (16), l'ouverture (11) vers l'extérieur est prévue de l'autre côté du corps de soupape (17).

5. Seringue selon l'une des revendications 1 à 4, caractérisée par le fait que le bord de l'extrémité du cylindre de seringue (1) du côté de l'espace extérieur est réalisé sous la forme d'une butée (20) qui empêche les pistons de seringue (5, 8) de sortir du cylindre de seringue (1).

**Claims**

1. A syringe for medical purposes comprising a syringe cylinder (1) designed at one end for the attachment of an injection needle (2), a syringe plunger (5) which is displaceable in the syringe cylinder (1) and which is fixedly carried on a plunger rod (6) which projects out of the syringe cylinder (1) at the end of the cylinder which is opposite to the needle attachment end, and a further syringe plunger (8) which is arranged in the syringe cylinder (1) on the side of the syringe plunger remote from the needle attachment end and which has the plunger rod (6) passing therethrough and which, besides being displaceable in the syringe cylinder (1), is also displaceable on the plunger rod (6), wherein the plunger rod (6) has a longitudinal duct (9) which extends within the plungter rod (6) over the region of displacement of the syringe plunger (8) which is displaceable on the plunger rod (6), the longitudinal duct opening on the one hand within the syringe cylinder (1) between the two syringe plungers (5, 8) adjacent the syringe plunger (5) which is fixed on the plunger rod (6), and on the other hand in the space outside the syringe cylinder (1) beyond the syringe plunger (8) which is displaceable on the plunger rod (6), characterised in that the longitudinal duct (9) can be closed by a non-return valve (12) which opens the longitudinal duct (9) only in the direction from the intermediate space (13) between the two syringe plungers (5, 8) to the space outside the syringe cylinder (1), against a spring force (18).

2. A syringe according to claim 1 characterised in that the longitudinal duct (9) terminates at its end towards the outside of the syringe in a valve chamber (15) which is provided in the plunger rod (6) and which has an opening (11) towards the outside space and which is enlarged in relation to the longitudinal duct (9) and which at the opening of the longitudinal duct (9) forms a valve seat (16) for a valve member (17), the valve member (17) being displaceable in the valve chamber (15) and being urged against the valve seat (16) by the spring force (18).

3. A syringe according to claim 2 characterised in that the valve chamber (15) is formed by a blind hole which is coaxial with respect to the longitudinal duct (9) and which opens at the free end of the plunger rod and which is closed outwardly by a plug (9), a coil spring for producing the spring force (18) being inserted between the plug (19) and the valve member (17).

4. A syringe according to claim 2 or claim 3 characterised in that the valve member (17) is provided laterally with free clearance relative to the wall of the valve chamber (15) and the opening (11) leading to the outside is provided on the other side of the valve member (17), as viewed from the valve seat (16).

5. A syringe according to one of claims 1 to 4 characterised in that the edge of the end of the syringe cylinder (1), which is towards the outside, is in the form of a stop (20) for preventing the syringe plungers (5, 8) from coming out of the syringe cylinder (1).

EP 0 254 765 B1

Fig.1

Fig.2

Fig.3